(19) Europäisches Patentamt
European Patent Office
Office européen des brevets

(11) Veröffentlichungsnummer : **0 301 444 B1**

(12) **EUROPÄISCHE PATENTSCHRIFT**

(45) Veröffentlichungstag der Patentschrift : **17.04.91 Patentblatt 91/16**

(51) Int. Cl.[5] : **C07C 45/68,** C07C 49/80, C07C 49/84, C07C 45/45, C07C 323/09

(21) Anmeldenummer : **88111896.2**

(22) Anmeldetag : **23.07.88**

(54) **Verfahren zur Herstellung von Arylperfluoralkylketonen.**

(30) Priorität : **29.07.87 DE 3725126**

(43) Veröffentlichungstag der Anmeldung : **01.02.89 Patentblatt 89/05**

(45) Bekanntmachung des Hinweises auf die Patenterteilung : **17.04.91 Patentblatt 91/16**

(84) Benannte Vertragsstaaten : **BE CH DE FR GB IT LI NL**

(56) Entgegenhaltungen :
**EP-A- 0 074 057**
**DE-C- 1 127 340**
**JOURNAL OF FLUORINE CHEMISTRY, Band 18, 1981, Seiten 117-129, Lausanne, CH; L.S. CHEN etal.: "Fluoro-ketones IV. Synthesis of phenylperfluoroalkyl ketones - mechanism of reaction between phenyllithium and fluoroesters"**

(56) Entgegenhaltungen :
**BULLETIN OF THE CHEMICAL SOCIETY OF JAPAN, Band 48, Nr. 4, 1975, Seiten 1339, 1340, Tokyo, JP; N. ISHIKAWA et al.: "The preparation of aryl heptafluoroisopropyl ketones from aroyl chlorides and hexafluoropropene in the presence of fluoride ions"**

(73) Patentinhaber : **HOECHST AKTIENGESELLSCHAFT**
**Postfach 80 03 20**
**W-6230 Frankfurt am Main 80 (DE)**

(72) Erfinder : **Kruse, Alfred, Dr.**
**Hornauer Strasse 199**
**W-6233 Kelkheim (Taunus) (DE)**
Erfinder : **Siegemund, Günter, Dr.**
**Frankfurter Strasse 21**
**W-6238 Hofheim am Taunus (DE)**
Erfinder : **Ruppert, Ingo, Dr.**
**verstorben (DE)**
Erfinder : **Schlich, Klaus**
**Falterer Pfad 13**
**W-5480 Remagen (DE)**

**EP 0 301 444 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Arylperfluoralkylketonen durch Umsetzung von aromatischen Carbonsäurederivaten mit Perfluoralkylhalogeniden in Gegenwart von Phosphorigsäuretriamiden.

Arylperfluoralkylketone sind wichtige Zwischenprodukte für die Herstellung von Arzneimitteln, Pflanzenschutzmitteln und Kunststoffen. Sie lassen sich auf den beiden folgenden allgemeinen Synthesewegen erzeugen.

Die Umsetzung von Perfluoralkylcarbonsäurehalogeniden mit Aromaten nach Art einer Friedel-Crafts-Substitution liefert die entsprechenden Ketone (Tetrahedron 42, 547-552 (1986)). Die Position, in der die Perfluoralkylcarbonylgruppe am aromatischen Ring gebunden ist, ist von Natur und Stellung vorhandener Substituenten abhängig, so daß sich mit diesem Verfahren überhaupt nur bestimmte Substitutionstypen von Arylperfluoralkylketonen erhalten lassen. Weitere Nachteile dieses Verfahrens sind die mögliche Bildung von Stellungsisomeren bei der Substitution am Arylring, eine mögliche mehrfache Substitution, die Beschränkung auf aktivierte Aromaten und auf bestimmte Substituenten am aromatischen Ring insofern, als manche Substituenten mit dem Friedel-Crafts-Katalysator reagieren oder Komplexe bilden und so eine weitere Reaktion im gewünschten Sinne unterbunden wird.

Es ist weiterhin bekannt, daß sich Arylperfluoralkylketone durch Umsetzung von Arylorganometallverbindungen mit Perfluoralkylcarbonsäurederivaten oder durch das umgekehrte Verfahren einer Umsetzung von aromatischen Carbonsäurederivaten mit Perfluoralkylorganometallverbindungen gewinnen lassen (J. Fluorine Chem. 18, 117-129 (1981) ; J. Am. Chem. Soc. 78, 2268-2270 (1956) ; Bull. Chem. Soc. Jap. 48, 1339-40 (1975)). Verwendet werden dabei Organometallverbindungen unedler Metalle, die gewöhnlich aus entsprechenden Arylhalogeniden bzw. Perfluoralkylhalogeniden und einem Metall, wie Magnesium, Lithium, Natrium, hergestellt werden.

Hier sind die in der Regel aufwendige Herstellung und die Labilität der zunächst herzustellenden Organometallverbindungen von Nachteil. Bei dem laut Literaturangaben am häufigsten durchgeführten allgemein anwendbaren Verfahren zur Herstellung von Arylperfluoralkylketonen werden 3 Mol Arylorganometallverbindung pro Mol Perfluoralkylcarbonsäure benötigt und zudem oft nur unbefriedigende Ausbeuten erzielt (J. Fluorine Chem. 18, 323-29 (1981)).

Die Erfindung bezieht sich nun auf ein einfaches, einstufiges Verfahren zur Herstellung von Arylperfluoralkylketonen aus aromatischen Carbonsäurederivaten unter Vermeidung der Herstellung und Verwendung der zuvor angeführten Organometallverbindungen.

Es wurde nun gefunden, daß sich Arylcarbonsäurederivate der allgemeinen Formel II (s. Patentanspruch 1), worin X als einwertiges Symbol Chlor, Brom oder Fluor und als zweiwertiges Symbol das Sauerstoffatom einen Anhydridbrücke bedeutet, mit Perfluoralkylhalogeniden der allgemeinen Formel $C_nF_{2n+1}$ –Hal (III), worin Hal Chlor, Brom oder Jod ist, bereits in Gegenwart von Phosphorigsäuretrisdialkylamiden (in anderen Worten Tris(dialkylamino)phosphanen) der allgemeinen Formel $P(N(Alkyl)_2)_3$ (IV) zu den gesuchten Arylperfluoralkylketonen der allgemeinen Formel I (s. Patentanspruch 1) umsetzen lassen. In den Formeln I und II stellen die Reste $R^1$ bis $R^5$ Wasserstoff dar oder haben die weiter unten angegebene Bedeutung.

Das erfindungsgemäße Verfahren hat nicht nur den Vorteil der Einfachheit, sondern auch den, daß die Ausgangsmaterialien gut zugänglich sind. Die Ausbeuten sind in vielen Fällen deutlich höher als entsprechende Literaturangaben für die nach anderen Methoden hergestellten Ketone.

Als aromatische Carbonsäurederivate (II) können die Halogenide (X = Chlor, Brom, Fluor) oder auch entsprechende Anhydride der aromatischen Carbonsäuren eingesetzt werden ; bevorzugt sind die, in der Regel auch am besten zugänglichen, Carbonsäurechloride und -fluoride. Die aromatischen Carbonsäurederivate können unsubstituiert sein oder einen oder mehrere gleiche oder unterschiedliche Substituenten $R^1$ bis $R^5$ mit anderer Bedeutung als Wasserstoff tragen. Geeignete Substituenten sind z.B. Alkyl-, Alkoxy- und Alkylthioreste jeweils mit 1 bis 6, insbesondere 1 bis 3 Kohlenstoffatomen, wobei die Alkylreste perfluoriert sein können, sowie Halogen (Fluor, Chlor, Brom, Jod). Zweckmäßig sind an den aromatischen Ring nicht mehr als drei und vorzugsweise höchstens zwei Substituenten $R^1$ bis $R^5$ mit anderer Bedeutung als Wasserstoff gebunden. Die Alkyl-, Alkoxy- und Alkylthiosubstituenten enthalten zweckmäßig zusammen höchstens 4 C-Atome und können geradkettig oder verzweigt sein.

Als Perfluoralkylhalogenide werden im allgemeinen Verbindungen mit 1 bis 6, insbesondere 1 bis 3 C-Atomen verwendet, die geradkettig oder verzweigt sein können, vorzugsweise $CF_3Br$ und die homologen Perfluoralkyljodide der allgemeinen Formel $C_nF_{2n+1}J$ mit n = 1 bis 6 bzw. bis 3.

Als Phosphorigsäuretrisdialkylamide (IV) kommen vor allem die niederen Alkylverbindungen in Frage, insbesondere solche mit $C_1$-$C_4$-Alkyl, wie Trisdimethylaminophosphan, Trisdiäthylaminophosphan und Trisdipropyl- bzw. -isopropylaminophosphan ; bevorzugt wird Trisdiäthylaminophosphan $P(N(CH_2CH_3)_2)_3$ verwendet. Dieses läßt sich sehr einfach in hohen Ausbeuten durch Reaktion von Phosphortrichlorid mit Diäthylamin in

einem gegenüber den Reaktionsteilnehmern inerten Lösungsmittel, z.B. einem aliphatischen, cycloaliphatischen oder aromatischen Kohlenwasserstoff oder einem Kohlenwasserstoffgemisch, erzeugen. Die Dialkylaminogruppen können gleiche oder verschiedene Alkylgruppen enthalten.

Bei der Umsetzung des Arylcarbonsäurehalogenids mit einem Perfluoralkylhalogenid wird unter dem Einfluß des Phosphorigsäuretrisdialkylamids formal ein Mol Halogen bzw. gemischtes Halogen abgespalten und ein salzartiges Addukt aus dem Phosphorigsäuretriamid und Halogen sowie ein Arylperfluoralkylketon gebildet.

Die Umsetzung von Arylcarbonsäurehalogenid mit Perfluoralkylhalogenid in Gegenwart vom Phosphorigsäuretriamid wird im allgemeinen bei Temperaturen von etwa − 100°C bis + 40°C durchgeführt. Die kurzkettigen Perfluoralkylhalogenide reagieren meist schon sehr rasch bei − 78°C. Bei den Perfluoralkylhalogeniden mit mindestens 2 C-Atomen ist es oft notwendig, die Reaktionstemperaturen zu erhöhen, um eine zügige Umsetzung zu erreichen ; bevorzugt werden dann Temperaturen oberhalb − 40°C und z.B. bis + 20°C. Die Reaktionsdauer ist bekanntlich von den übrigen Bedingungen, insbesondere der Reaktionstemperatur, abhängig. Die Reaktion ist im allgemeinen im Zeitraum von wenigen Minuten bis zu mehreren Stunden beendet.

Die Umsetzungen werden im allgemeinen ohne Anwendung von Überdruck ausgeführt. Jedoch kann es in einzelnen Fällen, z.B. bei der Umsetzung der Perfluormethylhalogenide, zweckmäßig sein, auch bei erhöhtem Druck zu arbeiten, vor allem wenn oberhalb der Siedetemperatur (bei Normaldruck) des Perfluoralkylhalogenids gearbeitet wird. Praktisch wird man dann also bei mindestens dem Eigendruck arbeiten.

Zweckmäßig wird das vorliegende Verfahren unter wasserfreien Bedingungen in Gegenwart eines gegenüber den Reaktionsteilnehmern inerten Lösungs- oder Verdünnungsmittels durchgeführt. Als solche kommen vor allem aprotische Flüssigkeiten zum Einsatz. Verwendet werden z.B. halogenierte Kohlenwasserstoffe wie Methylenchlorid, Tetrachloräthan, Nitrile, z.B. Acetonitril oder dessen Homologe oder Benzonitril, Ester wie Diäthylcarbonat oder Äthylencarbonat und Äther wie Tetrahydrofuran oder Dimethoxyäthan. Das Lösungsmittel sollte möglichst wasserfrei sein.

Es ist von Vorteil, während der gesamten Dauer der Reaktion, z.B. durch Rühren, für eine gute Durchmischung des Ansatzes zu sorgen sowie durch Wahl eines geeigneten Lösungsmittels die salzartigen Zwischen- und Begleitprodukte in Lösung zu halten.

Die Reagenzien werden in einer zum aromatischen Carbonsäurederivat mindestens äquivalenten Menge, oft in einem Überschuß von z.B. 2% bis 20% verwendet.

Die Art und die Reihenfolge der Vereinigung der drei Komponenten ist variierbar. Das erfindungsgemäße Verfahren kann beispielsweise so durchgeführt werden, daß man Lösungsmittel, Carbonsäurehalogenid und Perfluoralkylhalogenid vorlegt und das Phosphorigsäuretriamid zudosiert. Es können auch alle Komponenten gleichzeitig vereinigt werden.

Die Aufarbeitung des Reaktionsgemisches erfolgt vorteilhaft durch destillative Trennung der Komponenten. Oft ist es auch zweckmäßig, das entstandene Arylperfluoralkylketon durch eine Extraktion vom gleichzeitig entstandenen Phosphorigsäuretriamid-Halogen-Addukt (in anderen Worten ein Phosphoniumsalz) abzutrennen. Bei der Zugabe eines unpolaren Lösemittels, z.B. eines Kohlenwasserstoffs wie Hexan, zum Reaktionsgemisch erhält man zwei Phasen, wobei sich in der oberen Phase die Perfluoralkylverbindung befindet und die untere Phase im wesentlichen das als Begleitprodukt anfallende, im Kohlenwasserstoff nicht lösliche Phosphoniumsalz enthält.

**Beispiele**

1) In einem Rundkolben werden unter Schutzgas bei ca. − 70°C zu einer Lösung von 42 g (0,25 mol) 3,4-Dimethylbenzoesäurechlorid in 150 ml $CH_2Cl_2$ 41 g (0,27 mol) Trifluormethylbromid einkondensiert. Dann wird bei ca. − 70°C eine Lösung von 66,7 g (0,27 mol) Phosphorigsäuretrisdiäthylamid in 50 ml $CH_2Cl_2$ zudosiert, wobei die ersten 90% der Lösung rasch zugegeben und die letzten 10% der Lösung langsam (etwa innerhalb von 2 Stunden) zudosiert werden. Anschließend wird bei dieser Temperatur noch eine weitere Stunde gerührt und dann auf Raumtemperatur erwärmt. Nach Zugabe der gleichen Volumenmenge an Hexan bilden sich 2 Phasen. Nach Phasentrennung wird die untere Phase sorgfältig mit Hexan extrahiert ; die vereinigten Hexanphasen werden eingeengt und unter vermindertem Druck destilliert. Es werden 20,8 g (41% Ausbeute) an 1-(3,4-Dimethylphenyl)-2, 2, 2-trifluoräthanon-1 vom Kp. 98-99°C/22 mbar erhalten.

2) In einem Rundkolben werden unter Schutzgas bei ca. − 20°C 39 g (0,25 mol) m-Toluylsäurechlorid in 150 ml $CH_2Cl_2$ vorgelegt. Es werden zunächst 67 g (0,27 mol) Pentafluoräthyljodid einkondensiert und dann 66,7 g (0,27 mol) Phosphorigsäuretrisdiäthylamid zudosiert. Anschließend wird das Reaktionsgemisch noch 5 Stunden bei 0°C gerührt. Die Aufarbeitung analog zu Beispiel 1 liefert 33,2 g (56% Ausbeute) an 1-(3-Methylphenyl)-2, 2, 3, 3, 3-pentafluorpropanon-1 vom Kp 75-78°C/12 mbar.

3) In einem Rundkolben werden unter Schutzgas 31,5 g (0,185 mol) 3-Methoxybenzoylchlorid in 150 ml

$CH_2Cl_2$ vorgelegt. Bei ca. – 10°C werden 48,6 g (0,2 mol) Phosphorigsäuretrisdiäthylamid zugegeben. Bei 0°C werden dann 50 g (0,2 mol) Pentafluoräthyljodid innerhalb einer Stunde in das Reaktionsgemisch eingeleitet. Es wird weitere 5 Stunden bei 0°C gerührt und dann analog zu Beispiel 1 aufgearbeitet. Man erhält 22,9 g (61% Ausbeute) an 1-(3-Methoxyphenyl)-2, 2, 3, 3, 3-pentafluorpropanon-1 vom Kp 95-97°C/11 mbar.

4) In einem Rundkolben werden unter Schutzgas 22 g (0,18 mol) Benzoesäurefluorid und 55,3 g (0,18 mol) Perfluorisopropyljodid in 150 ml $CH_2Cl_2$ vorgelegt. Bei ca. – 70°C werden 45 g (0,18 mol) Phosphorigsäuretrisdiäthylamid zugetropft. Es wird 12 Stunden bei – 70°C gerührt und nach dem Erwärmen analog zu Beispiel 1 aufgearbeitet. Man erhält 38,5 g (78%) 1-Phenyl-2, 3, 3, 3-tetrafluor-2-trifluormethyl-propanon-1 vom Kp 90-92°C/80 mbar.

5) In einem Rundkolben werden unter Schutzgas 27,3 g (0,16 mol) p-Methoxybenzoylchlorid und 53,3 g (0,18 mol) Perfluorisopropyljodid in 150 ml $CH_2Cl_2$ vorgelegt. Bei ca. – 20°C werden 45 g (0,18 mol) Phosphorigsäuretrisdiäthylamid innerhalb von 3 Stunden zudosiert. Anschließend wird 5 Stunden bei ca. – 10°C gerührt und nach dem Erwärmen analog zu Beispiel 1 aufgearbeitet. Es werden 37 g (76%) 1-(4-Methoxyphenyl)-2, 3, 3, 3-tetrafluor-2-trifluormethylpropanon-1 vom Kp 97-99°C/10 mbar erhalten.

6) In einem Rundkolben werden unter Schutzgas bei ca. – 20°C 35 g (0,25 mol) Benzoylchlorid in 150 ml $CH_2Cl_2$ vorgelegt. Es werden zunächst 67 g (0,27 mol) Pentafluoräthyljodid einkondensiert und dann 66,7 g (0,27 mol) Phosphorigsäuretrisdiäthylamid zudosiert. Anschließend wird das Reaktionsgemisch noch 5 Stunden bei 0°C gerührt. Nach Zugabe der gleichen Volumenmenge an Hexan zum Reaktionsgemisch bilden sich 2 Phasen. Nach Phasentrennung wird die untere Phase sorgfältig mit Hexan extrahiert ; die vereinigten Hexanphasen werden eingeengt und unter vermindertem Druck destilliert. Es werden 20,8 g (58% Ausbeute) an Pentafluoräthylphenylketon vom Kp. 76-77/40 mbar erhalten.

7) bis 16) Die Herstellung der folgenden Verbindungen ergibt sich aus der nachstehenden Tabelle, wobei R den oder die angegebenen Reste $R^1$ bis $R^5$ der Formel I darstellt. In Beispiel 16 wurde analog Beispiel 2 gearbeitet, jedoch mit der weiteren Abweichung, daß als Lösungsmittel Acetonitril anstelle von Dichlormethan verwendet wurde.

| Beispiel | R | $R_F$ | Ausbeute | $K_p$ °C/mbar | Verfahren analog Beispiel |
|---|---|---|---|---|---|
| 7 | H | $CF_3$ | 52 % | 84-85/80 | 1 |
| 8 | 3-$CH_3$ | $CF_3$ | 48 % | 75-76/20 | 1 |
| 9 | 4-$CH_3$ | $C_2F_5$ | 58 % | 82-83/10 | 2 |
| 10 | 3,5$(OCH_3)_2$ | $C_2F_5$ | 44 % | 73-74/0,2 | 2 |
| 11 | 2-Cl | $C_3F_7$ | 83 % | 76-77/7 | 4 |
| 12 | 4-Cl | $C_2F_5$ | 42 % | 88-89/10 | 2 |
| 13 | 3-F | $C_3F_7$ | 81 % | 70-71/20 | 4 |
| 14 | 3-F | $C_2F_5$ | 21 % | 95-97/90 | 2 |
| 15 | 4-$CF_3$ | $C_2F_5$ | 42 % | 86-87/20 | 2 |
| 16 | 3-$CH_3$ | $C_2F_5$ | 44 % | 78-79/9 | 2 |

## Ansprüche

1. Verfahren zur Herstellung von Arylperfluoralkylketonen der allgemeinen Formel I

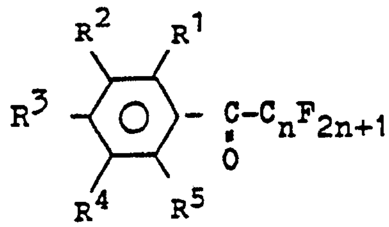

(I),

worin die Reste $R^1$ bis $R^5$ gleich oder verschieden sind und Wasserstoff, Alkyl mit 1 bis 6 Kohlenstoffatomen, das perfluoriert sein kann, Halogen, Alkoxy oder Alkylthio mit jeweils 1 bis 6 Kohlenstoffatomen im Alkylrest sind und n 1 bis 6 ist, dadurch gekennzeichnet, daß man Arylcarbonsäurederivate der allgemeinen Formel II

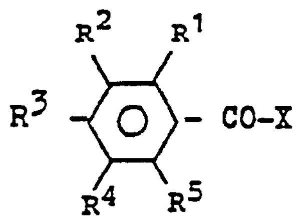

(II),

worin $R^1$ bis $R^5$ die angegebene Bedeutung haben und X als einwertiges Symbol Chlor, Brom oder Fluor und als zweiwertiges Symbol das Sauerstoffatom einen Anhydridbrücke bedeutet, mit Perfluoralkylhalogeniden der allgemeinen Formel $C_nF_{2n+1}$-Hal (III), worin Hal Chlor, Brom oder Jod ist und n die angegebene Bedeutung hat, in Gegenwart von Phosphorigsäuretrisdialkylamiden der allgemeinen Formel $P[N(Alkyl)_2]_3$ (IV) umsetzt.

2. Verfahren nach Anspruch 1, dadurch gekennzeichnet, daß an den aromatischen Ring nicht mehr als 3 und vorzugsweise nur höchstens zwei Substituenten $R^1$ bis $R^5$ mit anderer Bedeutung als Wasserstoff gebunden sind.

3. Verfahren nach Anspruch 1 oder 2, dadurch gekennzeichnet, daß die Alkyl-, Alkoxy- und Alkylthiosubstituenten zusammen höchstens 4 C-Atome enthalten.

4. Verfahren nach einem oder mehreren der Ansprüche 1 bis 3, dadurch gekennzeichnet, daß die Umsetzung bei Temperaturen von etwa – 100°C bis + 40°C durchgeführt wird.

5. Verfahren nach Anspruch 4, dadurch gekennzeichnet, daß Perfluoralkylhalogenide mit mindestens 2 C-Atomen bei einer Temperatur oberhalb – 40°C, zweckmäßig bis + 20°C, umgesetzt werden.

6. Verfahren nach einem oder mehreren der Ansprüche 1 bis 5, dadurch gekennzeichnet, daß die Umsetzung ohne Anwendung von Überdruck erfolgt.

7. Verfahren nach einem oder mehreren der Ansprüche 1 bis 5, dadurch gekennzeichnet, daß Perfluormethylhalogenide bei höherem als Atmosphärendruck umgesetzt werden.

8. Verfahren nach einem oder mehreren der Ansprüche 1 bis 7, dadurch gekennzeichnet, daß die Umsetzung unter wasserfreien Bedingungen in Gegenwart eines gegenüber den Reaktionsteilnehmern inerten Lösungs- oder Verdünnungsmittels, vorteilhaft einer aprotischen Flüssigkeit, durchgeführt wird.

9. Verfahren nach einem oder mehreren der Ansprüche 1 bis 8, dadurch gekennzeichnet, daß die anderen Reaktionsteilnehmer in einer zum Arylcarbonsäure derivat II mindestens äquivalenten Menge und höchstens in einem Überschuß bis zu 20% eingesetzt werden.

10. Verfahren nach einem oder mehreren der Ansprüche 1 bis 9, gekennzeichnet durch wenigstens eines der Merkmale, daß

a) X in Formel II Fluor oder Chlor ist,

b) das Perfluoralkylhalogenid III 1 bis 3 C-Atome hat, vorzugsweise aber $CF_3Br$ oder ein Perfluoralkyljodid ist und

c) im Phosphorigsäuretrisdialkylamid IV das Alkyl 1 bis 4 C-Atome hat, vorzugsweise aber Äthyl ist.

## Claims

1. A process for the preparation of arylperfluoralkyl ketones of the general formula I

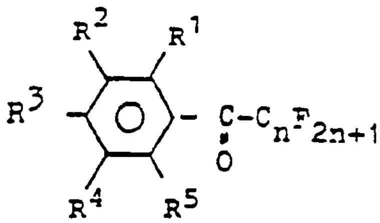

(I),

wherein the radicals $R^1$ to $R^5$ are equal or different and represent at least one of the substituents hydrogen, halogen, alkyl, alkoxy, alkylthio and perfluorinated alkyl, each having from 1 to 6 carbon atoms and n is an integer from 1 to 6, which comprises reacting a derivative of an aryl carboxylic acid having the general formula II

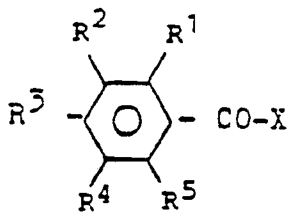

(II),

wherein $R^1$ to $R^5$ have the above-mentioned meaning and X as a monovalent moiety represents fluorine, chlorine or bromine and as a bivalent moiety the oxygen atom of an anhydride bridge, with a perfluoroalkyl halide of the general formula $C_nF_{2n+1}$-Hal (III), wherein Hal represents chlorine, bromine or iodine and n has the above-mentioned meaning in the presence of a trisdialkyl amide of phosphorous acid of the general formula $P[(alkyl)_2]_3$(IV).

2. A process as claimed in claim 1, wherein there are bound to the aromatic ring not more than three, preferably at most two of the substituents $R^1$ to $R^5$ which have a meaning other than hydrogen.

3. A process as claimed in claim 1 or 2, wherein the alkyl, alkoxy and alkylthio substituents $R^1$ to $R^5$ contain altogether at most 4 carbon atoms.

4. A process as claimed in one or more of claims 1 to 3, wherein the reaction is carried out at a temperature in the range of from about − 100°C to + 40°C.

5. A process as claimed in claim 4, wherein a perfluoroalkyl halide of at least 2 carbon atoms is reacted at a temperature above − 40°C, preferably at a temperature of up to + 20°C,

6. A process as claimed in one or more of claims 1 to 5, which is carried out at a pressure not exceeding ambient pressure.

7. A process as claimed in one or more of claims 1 to 5, wherein a perfluoromethyl halide is reacted at a pressure higher than ambient pressure.

8. A process as claimed in one or more of claims 1 to 7, which is carried out under anhydrous conditions in the presence of a solvent or diluent inert towards the reactants, preferably in the presence of an aprotic liquid.

9. A process as claimed in one or more of claims 1 to 8, wherein the reactant other than the arylcarboxylic acid derivative II are applied in an amount at least equivalent to the aryl carboxylic acid derivative, and at most in an amount of up to 20% above the equivalent amount.

10. A process as claimed in one or more of claims 1 to 9, wherein at least one of the following features is applied

a) X in formula II represents fluorine or chlorine,

b) the perfluoralkyl halide III has from 1 to 3 carbon atoms, but is preferably $CF_3Br$ or a perfluoroalkyl iodide and

c) the alkyl in the trisdialkylamide of phosphorous acid IV has from 1 to 4 carbon atoms, but is preferably ethyl.

## Revendications

1. Procédé pour préparer des (aryl)-(perfluoralkyle)cétones répondant à la formule générale I :

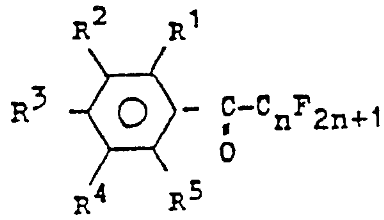

(I)

dans laquelle les symboles $R^1$ à $R^5$ représentent chacun, indépendamment des uns des autres, l'hydrogène, un alkyle contenant de 1 à 6 atomes de carbone, qui peut être perfluoré, un halogène, ou un radical alcoxy ou alkylthio dont la partie alkyle contient de 1 à 6 atomes de carbone, et n désigne un nombre de 1 à 6, procédé caractérisé en ce qu'on fait réagir des dérivés d'acides aryl-carboxyliques répondant à la formule II :

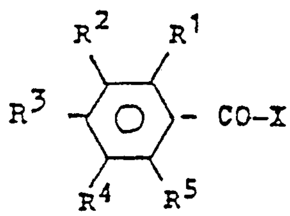

(II)

dans laquelle $R^1$ à $R^5$ ont les significations qui leur ont été données et X, en tant que symbole univalent, représente le chlore, le brome ou le fluor et, en tant que symbole bivalent, l'atome d'oxygène d'un pont anhydride, avec des halogénures de perfluoralkyles répondant à la formule générale III :

$$C_nF_{2n+}\text{-Hal} \quad (III)$$

dans laquelle Hal représente le chlore, le brome ou l'iode et n a la signification indiquée, en présence de tris-(dialkylamides) de l'acide phosphoreux de formule générale :

$$P[N(alkyl)_2]_3 \quad (IV)$$

2. Procédé selon la revendication 1, caractérisé en ce que le noyau aromatique porte au plus 3, de préférence au plus 2, substituants $R_1$ à $R_5$ ayant une signification autre que l'hydrogène.

3. Procédé selon l'une des revendications 1 et 2, caractérisé en ce que les substituants alkyles, alcoxy et alkylthio contiennent, ensemble, au plus 4 atomes de carbone.

4. Procédé selon l'une quelconque des revendications 1 à 3, caractérisé en ce que la réaction est effectuée à des températures d'environ – 100 à + 40°C.

5. Procédé selon la revendication 4, caractérisé en ce qu'on fait réagir des halogénures de perfluoralkyles à au moins 2 atomes de carbone à une température supérieure à – 40°C, avantageusement à une température pouvant aller jusqu'à + 20°C,

6. Procédé selon l'une quelconque des revendications 1 à 5, caractérisé en ce qu'on effectue la réaction sans application d'une surpression.

7. Procédé selon l'une quelconque des revendications 1 à 5, caractérisé en ce qu'on fait réagir des halogénures de perfluorométhyle sous une pression supérieure à la pression atmosphérique.

8. Procédé selon l'une quelconque des revendications 1 à 7, caractérisé en ce qu'on effectue la réaction dans des conditions anhydres, en présence d'un solvant ou diluant inerte à l'égard des corps qui prennent part à la réaction, avantageusement en présence d'un liquide aprotique.

9. Procédé selon l'une quelconque des revendications 1 à 8, caractérisé en ce que les autres partenaires réactionnels sont mis en jeu en une quantité au moins équivalente par rapport à la dérivé d'acide aryl-carboxylique II et représentant au plus un excès de 20%.

10. Procédé selon l'une quelconque des revendications 1 à 9, caractérisé par au moins une des particularités suivantes :

a) X dans la formule II représente le fluor ou le chlore,
b) l'halogénure de perfluoralkyle IIIcontient de 1 à 3 atomes de carbone, mais est de préférence $CF_3Br$ ou un iodure de perfluoralkyle,
c) dans le tris-(dialkylamide) de l'acide phosphoreux (IV) l'alkyle contient de 1 à 4 atomes de carbone, mais est de préférence un radical éthyle.